(19) Europäisches Patentamt
European Patent Office
Office européen des brevets

(11) **EP 2 381 992 B1**

(12) **EUROPEAN PATENT SPECIFICATION**

(45) Date of publication and mention
of the grant of the patent:
**27.02.2019 Bulletin 2019/09**

(21) Application number: **10730997.3**

(22) Date of filing: **14.01.2010**

(51) Int Cl.:
*A61M 16/00* (2006.01)          *A61B 5/0488* (2006.01)
*A61B 5/08* (2006.01)

(86) International application number:
**PCT/CA2010/000043**

(87) International publication number:
**WO 2010/081223 (22.07.2010 Gazette 2010/29)**

(54) **DEVICE FOR DETERMINING A LEVEL OF VENTILATORY ASSIST TO A PATIENT**

VORRICHTUNG ZUR BESTIMMUNG EINES BEATMUNGSUNTERSTÜTZUNGSNIVEAUS FÜR EINEN PATIENTEN

DISPOSITIF DE DÉTERMINATION D'UN NIVEAU D'ASSISTANCE VENTILATOIRE À UN PATIENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.01.2009 US 193987 P**

(43) Date of publication of application:
**02.11.2011 Bulletin 2011/44**

(73) Proprietor: **St. Michael's Hospital**
**Toronto, ON M5B 1W8 (CA)**

(72) Inventors:
• **SINDERBY, Christer**
**Toronto, Ontario M6S 1W6 (CA)**
• **BECK, Jennifer**
**Toronto, Ontario M6S 1W6 (CA)**

(74) Representative: **Ipside**
**7-9 Allées Haussmann**
**33300 Bordeaux Cedex (FR)**

(56) References cited:
EP-A1- 1 204 439          WO-A1-2007/082384
US-A- 3 890 967          US-A1- 2003 226 565
US-A1- 2009 159 082          US-B1- 7 021 310

• JADRANKA SPAHIJA ET AL: "Closed-Loop Control of Respiratory Drive Using Pressure-Support Ventilation", AMERICAN JOURNAL OF RESPIRATORY AND CRITICAL CARE MEDICINE, vol. 171, no. 9, 1 May 2005 (2005-05-01), pages 1009-1014, XP055162392, ISSN: 1073-449X, DOI: 10.1164/rccm.200407-856OC

**Description**

<u>FIELD</u>

The present invention is concerned with determining a level of ventilatory assist to be delivered a patient.

<u>BACKGROUND</u>

In practice, an adequate level of ventilatory assist to be delivered to a patient under mechanical ventilation is difficult to determine since unloading of the patient involves compensation for increased respiratory demand in terms of metabolic drive, resistive and elastic loads imposed by the patient's respiratory system, as well as weakness of the inspiratory muscles. There is therefore a need for a technique that facilitates such determination. As prior art, Document EP1204439 B1 discloses a method and a device capable of adjusting the degree of inspiratory assist in relation to the real need of the patient and to compensate for the degree of incapacity of the patient. The device of EP1204439 B1 determines a control signal to be input to a lung ventilator, said control signal being the result of multiplying a gain by a RMS value derived from measurement of patient neural activity as represented by EMG activity of the patient's diaphragm, wherein the gain factor is increased, decreased or left unchanged depending on a comparison of the present breath RMS value with a target drive signal quantified as, among others, the mean, he median, the total or the peak of the EMGdi signal; the lung ventilator further has a pressure sensor for providing feedback of actual respiratory pressure close to a patient interface, and the ventilator is controlled on the basis of a comparison between the control signal and the signal from the pressure sensor.

<u>SUMMARY</u>

The present invention is defined in the annexed claim 1.

In a comparative example not forming part of the invention, a method is herein described for determining a level of ventilatory assist to be delivered to a patient by a mechanical ventilator in response to a measure of a patient's neural respiratory drive multiplied by an amplification factor, comprising: calculating an existing predicted ventilatory assist pressure; measuring an existing resulting pressure delivered to the patient by the mechanical ventilator; changing the amplification factor from an existing amplification factor to a new amplification factor; calculating a new predicted ventilatory assist pressure using the new amplification factor; measuring a new resulting pressure delivered to the patient by the mechanical ventilator after the amplification factor has been changed from the existing amplification factor to the new amplification factor; comparing the new predicted ventilatory assist pressure and the existing predicted ventilatory assist pressure to determine an anticipated change in pressure that will be delivered to the patient by the mechanical ventilator; comparing the new resulting pressure and the existing resulting pressure to determine an actual change in pressure delivered to the patient by the mechanical ventilator; comparing the anticipated change in pressure with the actual change in pressure; and delivering a decision to increase, maintain or decrease the amplification factor in response to the comparison between the anticipated change and the actual change in pressure.

According to the invention, a device is provided for determining a level of ventilatory assist to be delivered to a patient by a mechanical ventilator in response to a measure of a patient's neural respiratory drive multiplied by an amplification factor, comprising: means for measuring an electrical activity of a respiratory related muscle of the patient as a representation of the patient's neural respiratory drive $EAdi_{old}$ ; a first calculator for multiplying the patient's neural respiratory drive $EAdi_{old}$ by an existing amplification factor $AF_{old}$ to obtain an existing predicted ventilatory assist pressure $P_{target\ old}$ ; a first sensor for sensing an existing resulting pressure $P_{result\ old}$ delivered to the patient by the mechanical ventilator; a computerized modifier of the amplification factor from the existing amplification factor $AF_{old}$ to a new amplification factor $AF_{new}$ ; a second calculator for multiplying the patient's neural respiratory drive $EAdi_{old}$ by the new amplification factor $EAdi_{old}$ to obtain a new predicted ventilatory assist pressure $P_{target\ new}$ ; a second sensor for sensing a new resulting pressure $P_{result\ new}$ delivered to the patient by the mechanical ventilator after the amplification factor has been changed from the existing amplification factor $AF_{old}$ to the new amplification factor $AF_{new}$ ; a first comparator of the new predicted ventilatory assist pressure $P_{target\ new}$ and the existing predicted ventilatory assist pressure $P_{target\ old}$ to determine an anticipated change in pressure $\Delta P_{target\ new-old}$ that will be delivered to the patient by the mechanical ventilator; a second comparator of the new resulting pressure $P_{result\ new}$ and the existing resulting pressure $P_{result\ old}$ to determine an actual change in pressure $\Delta P_{result\ new-old}$ delivered to the patient by the mechanical ventilator; a third comparator of the anticipated change in pressure and the actual change in pressure calculating a ratio $\Delta P_{result\ new-old}$ / $\Delta P_{target\ new-old}$ ; and a third calculator including a decision algorithm to take a decision as to increase, maintain or decrease the amplification factor depending on the value of the ratio $\Delta P_{result\ new-old}$ / $\Delta P_{target\ new-old}$ as calculated by the third comparator; wherein the modifier is adapted to automatically change the amplification factor as indicated by the decision from the decision algorithm of the third calculator; and wherein the first calculator, second calculator, first comparator, second comparator, third comparator and third calculator are implemented in a computer or computers.

## BRIEF DESCRIPTION OF THE DRAWINGS

The foregoing and other features of the method and device for determining a level of ventilatory assist will become more apparent from reading of the following non restrictive description of illustrative embodiments thereof and of comparative examples not falling within the scope of the invention, given by way of example only with reference to the accompanying drawings in which:

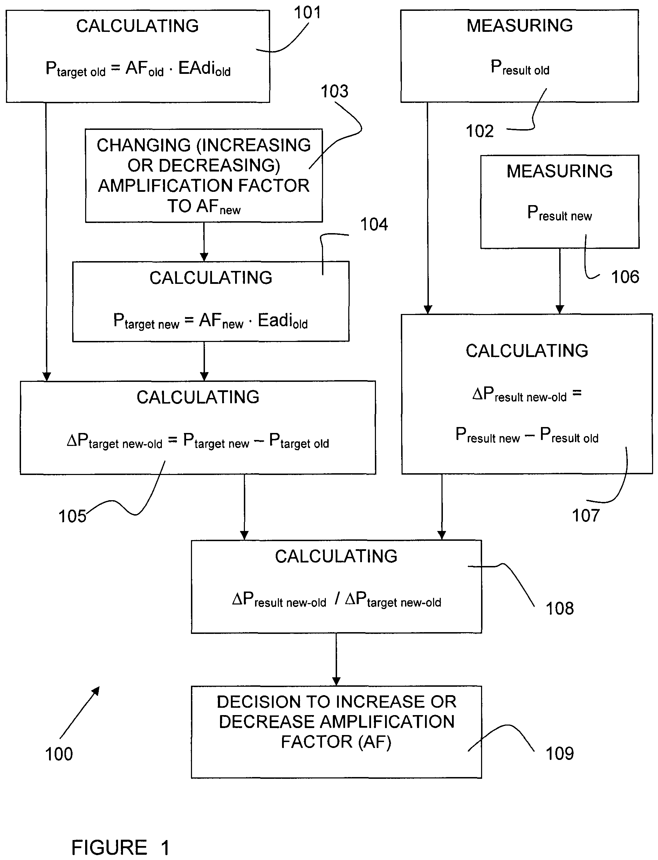

Figure 1 is a schematic flow chart of a method, according to a comparative example not falling within the scope of the invention, for determining the level of ventilatory assist to be delivered to the patient by the mechanical ventilator in response to the measure of the patient's neural respiratory drive multiplied by the amplification factor; and

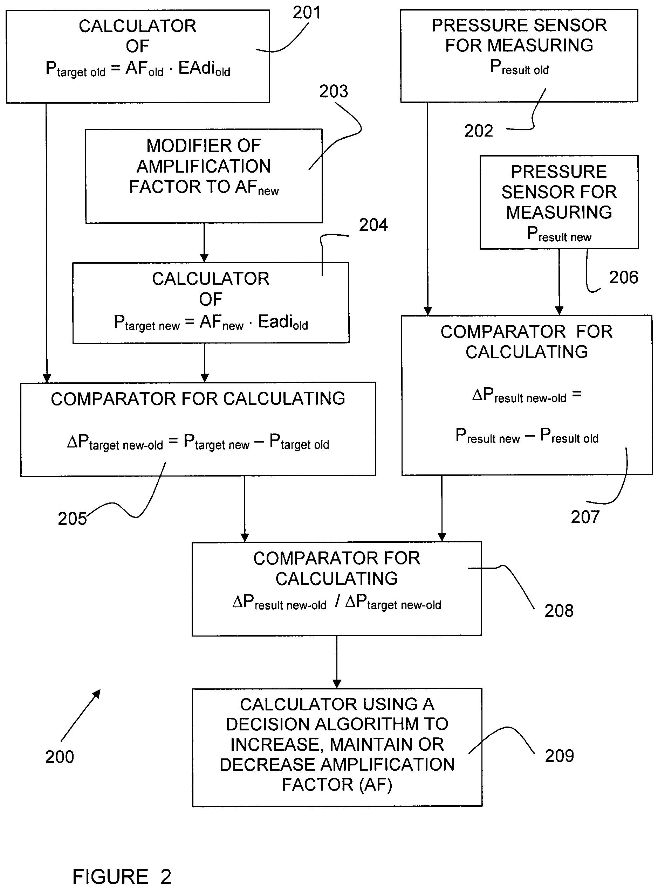

Figure 2 is a schematic block diagram of a device for determining the level of ventilatory assist to be delivered to the patient by the mechanical ventilator in response to the measure of the patient's neural respiratory drive multiplied by the amplification factor.

## DETAILED DESCRIPTION

The method, according to a comparative example not falling within the scope of the invention, and device for determining the level of ventilatory assist to be delivered to the patient by the mechanical ventilator in response to the measure of the patient's neural respiratory drive multiplied by the amplification factor will now be described.

The level of ventilatory assist to the patient is controlled by applying to the mechanical ventilator a control signal representative of a predicted ventilatory assist pressure $P_{target}$ determined as a function of the patient's neural respiratory drive and an amplification factor AF. The electrical activity of the patient's diaphragm EAdi is measured as the patient's neural respiratory drive and is multiplied by the amplification factor AF to describe the predicted ventilatory assist pressure $P_{target}$.

An example of technique for measuring the electrical activity of the patient's diaphragm EAdi is described in US Patent No. 5,671,752 (Sinderby et al.) issued on September 30, 1997. Although in the herein described embodiment the electrical activity of the patient's diaphragm EAdi is used as a representation of the patient's neural respiratory drive, it should be kept in mind that the electrical activity of another respiratory related muscle can be used as the representation of the patient's neural respiratory drive.

The method, according to a comparative example not falling within the scope of the invention, and device for determining the level of ventilatory assist to be delivered to the patient uses a prediction (predicted ventilatory assist pressure $P_{target}$) of the pressure to be delivered to the patient by the mechanical ventilator in response to a given change of the amplification factor AF and a comparison between the predicted ventilatory assist pressure $P_{target}$ and a resulting pressure $P_{result}$ actually delivered to the patient by the mechanical ventilator in response to the given change of the amplification factor AF. For example, if the measured electrical activity of the patient's diaphragm EAdi is 10 $\mu$V and the amplification factor AF is increased from 0 cm $H_2O/\mu V$ to 1 cm $H_2O/\mu V$, it can be expected that the predicted ventilatory assist pressure $P_{target}$ will be 10 cm $H_2O$. If the resulting pressure $P_{result}$ turns out to be 10 cm $H_2O$, this means that the patient has absorbed/accepted the whole ventilatory assist with no reduction of the patient's neural respiratory drive since the electrical activity of the patient's diaphragm EAdi remained unchanged. If, on the other hand, the electrical activity of the patient's diaphragm EAdi reduced from 10 $\mu$V to 1 $\mu$V, the resulting pressure $P_{result}$ becomes 1 cm $H_2O$ although the predicted ventilatory assist pressure $P_{target}$ was 10 cm $H_2O$, showing that the patient does not require the increase of ventilatory assist and rather down regulates his/her neural respiratory drive (EAdi).

If the patient already receives ventilatory assist and, for example, the amplification factor AF is 1 and the electrical activity of the patient's diaphragm EAdi is 10 $\mu$V, it is possible to calculate an expected increase of the predicted pressure $P_{target}$ if, for example, the amplification factor AF is increased to 1.5. The predicted ventilatory assist pressure will then be $P_{target}$ = 1.5 cm $H_2O/\mu V$ x 10 $\mu$V = 15 cm $H_2O$. If the increase of the amplification factor AF from 1.0 cm $H_2O/\mu V$ to 1.5 cm $H_2O/\mu V$ increases the resulting pressure $P_{result}$ from 10 cm $H_2O$ to 15 cm $H_2O$, this indicates that the patient welcome the increase of ventilatory assist (increase in pressure) and maintains his/her neural respiratory drive (EAdi).

If, on the other hand, the amplification factor AF is increased from 1.0 cm $H_2O/\mu V$ to 1.5 cm $H_2O/\mu V$ but the resulting pressure $P_{result}$ increases from 10 cm $H_2O$ to 12 cm $H_2O$ while the predicted ventilatory assist pressure $P_{target}$ indicates a 5 cm $H_2O$ increase, the patient does not welcome the whole increase in ventilatory assist and rather down regulates his/her neural respiratory drive (EAdi).

The method 100, according to a comparative example not falling within the scope of the invention, and device 200 for determining the level of ventilatory assist to be delivered to the patient by the mechanical ventilator in response to the measure of a patient's neural respiratory drive multiplied by the amplification factor will now be described with reference to Figures 1 and 2.

***Operation 101 (Figure 1)***

At a stable level of ventilatory assist, a calculator 201 (Figure 2) calculates an existing predicted ventilatory assist pressure $P_{target\ old}$ by multiplying the existing amplification factor $AF_{old}$ by the existing electrical activity of the patient's diaphragm $EAdi_{old}$, in accordance with the following relation:

$$P_{target\ old} = AF_{old} \cdot EAdi_{old} \tag{1}$$

***Operation 102 (Figure 1)***

At a stable level of ventilatory assist, a pressure sensor 202 (Figure 2) measures the existing resulting pressure $P_{resuit\ old}$ delivered to the patient by the mechanical ventilator. This can be made, for example, by measuring the pressure in the mechanical ventilator or mechanical ventilator circuit.
When the neurally controlled ventilator system has been operated at a given amplification factor for a certain time, the existing predicted pressure $P_{target\ old}$ and the existing resulting pressure $P_{result\ old}$ will assume very similar if not the same values.

***Operation 103 (Figure 1)***

A modifier 203 (Figure 2) of amplification factor, for example a keyboard, is used by the medical personnel, for example a caregiver, in a comparative example not falling within the scope of the invention, to change (increase or decrease) an existing amplification factor $AF_{old}$ to a new amplification factor $AF_{new}$.
According to the invention, the modifier 203 is implemented by an automatic computerized modifier requiring no intervention from the medical personnel.

***Operation 104 (Figure 1)***

A calculator 204 (Figure 2) calculates a new predicted ventilatory assist pressure $P_{target\ new}$ by multiplying the new amplification factor $AF_{new}$ by the existing electrical activity of the patient's diaphragm $EAdi_{old}$, in accordance with the following relation:

$$P_{target\ new} = AF_{new} \cdot EAdi_{old} \tag{2}$$

***Operation 105 (Figure 1)***

A comparator 205 (Figure 2) compares the new predicted ventilatory assist pressure $P_{target\ new}$ to the existing predicted ventilatory assist pressure $P_{target\ old}$ using, for example, the following relation:

$$\Delta P_{target\ new\text{-}old} = P_{target\ new} - P_{target\ old} \tag{3}$$

to show an anticipated change (increase or decrease) in pressure ($\Delta P_{target\ new\text{-}old}$) that will be delivered to the patient by the mechanical ventilator.

***Operation 106 (Figure 1)***

A pressure sensor 206 (Figure 2) measures the new resulting pressure $P_{result\ new}$ actually delivered to the patient by the mechanical ventilator following change from the existing amplification factor $AF_{old}$ to the new amplification factor $AF_{new}$. This can be made, for example, by measuring the pressure in the mechanical ventilator or mechanical ventilator circuit.

***Operation 107 (Figure 1)***

A comparator 207 (Figure 2) compares the new resulting pressure $P_{result\ new}$ to the existing resulting pressure

$P_{result\ old}$ using, for example, the following relation:

$$\Delta P_{result\ new\text{-}old} = P_{result\ new} - P_{result\ old} \qquad (4)$$

to show an actual change (increase or decrease) in pressure ($\Delta P_{result\ new\text{-}old}$) delivered to the patient by the mechanical ventilator.

**Operation 108 (Figure 1)**

A comparator 208 (Figure 2) compares the anticipated change (increase or decrease) in pressure $\Delta P_{target\ new\text{-}old}$ to the actual change (respectively increase or decrease) in pressure $\Delta P_{result\ new\text{-}old}$ using calculation of the following ratio:

$$\Delta P_{result\ new\text{-}old} / \Delta P_{target\ new\text{-}old} \qquad (5)$$

to express a relation between the anticipated change and actual change in the level of pressure delivered to the patient by the mechanical ventilator. The ratio $\Delta P_{result\ new\text{-}old} / \Delta P_{target\ new\text{-}old}$ will range between 0 and 1, such that the response to change (increase or decrease) of the amplification factor AF can be divided into classes as described hereinafter.

When the amplification factor is increased in operation 103 (Figure 1), a ratio $\Delta P_{result\ new\text{-}old} / \Delta P_{target}$ new-old equal to 1 suggests that the increase in ventilatory assist from the mechanical ventilator was welcomed by the patient, whereas a ratio $\Delta P_{result\ new\text{-}old} / \Delta P_{target\ new\text{-}old}$ of 0.5 suggests that only 50% of the increase in ventilatory assist delivered by the mechanical ventilator was welcomed by the patient and that the patient down regulated his/her neural respiratory drive (EAdi).

**Operation 109 (Figure 1)**

A calculator 209 (Figure 2) includes a decision algorithm implemented to take a decision as to increase or decrease the amplification factor AF for example as described hereinafter.

When the amplification factor AF is increased in operation 103 (Figure 1), the ratio $\Delta P_{result\ new\text{-}old} / \Delta P_{target\ new\text{-}old}$ is positive. For example, the decision can then be as follows:

- In response to a ratio $\Delta P_{result\ new\text{-}old} / \Delta P_{target\ new\text{-}old} = 0.8$ to 1, the decision algorithm of the calculator 209 (Figure 2) delivers a decision to further increase the amplification factor AF;

- In response to a ratio $\Delta P_{result\ new\text{-}old} / \Delta P_{target\ new\text{-}old} = 0.5$ to 0.8, the decision algorithm of the calculator 209 (Figure 2) delivers a decision to maintain the amplification factor AF unchanged; and

- In response to a ratio $\Delta P_{result\ new\text{-}old} / \Delta P_{target\ new\text{-}old.} < 0.5$, the decision algorithm of the calculator 209 (Figure 1) delivers a decision to decrease the amplification factor AF.

When the amplification factor AF is decreased in operation 103 (Figure 1), the ratio $\Delta P_{result\ new\text{-}old} / \Delta P_{target\ new\text{-}old}$ is negative. For example, the decision can then be as follows:

- In response to a ratio $\Delta P_{resut\ new\text{-}old} / \Delta P_{target\ new\text{-}old} = -0.8$ to -1, the decision algorithm of the calculator 209 (Figure 2) delivers a decision to increase the amplification factor AF;

- In response to a ratio $\Delta P_{result\ new\text{-}old} / \Delta P_{target\ new\text{-}old} = -0.5$ to -0.8, the decision algorithm of the calculator 209 (Figure 2) delivers a decision to maintain the amplification factor AF unchanged; and

- In response to a ratio $\Delta P_{result\ new\text{-}old} / \Delta P_{target\ new\text{-}old} > -0.5$, the decision algorithm of the calculator 209 (Figure 2) delivers a decision to further decrease the amplification factor AF.

If the decision algorithm of the calculator 209 (Figure 2) requests a change (increase or decrease) of the amplification factor, the complete, above described procedure including operations 101-109 is then repeated.

In operation 103 (Figure 1), the modifier 203 (Figure 2) of amplification factor will be used by the medical personnel, in a comparative example not falling within the scope of the invention, to change (increase or decrease) or, according to the invention, automatically changes (increase or decrease) the amplification factor AF as indicated. As a non limitative example, the modifier 203 (Figure 2) may be designed to automatically change the amplification factor AF as a function of the ratio $\Delta P_{\text{result new-old}} / \Delta P_{\text{target new-old}}$ according to a predetermined relation, for example by predetermined steps. According to a comparative example not falling within the scope of the invention, it can be left to the medical personnel to decide to what extent the amplification factor should be changed through the modifier 203 (Figure 2).

The calculator 201, calculator 204, comparator 205, comparator 207, comparator 208 and calculator 209 are implemented by a computer or computers, for example a single, suitably programmed general purpose computer.

**Claims**

1. A device for determining a level of ventilatory assist to be delivered to a patient by a mechanical ventilator in response to a measure of a patient's neural respiratory drive multiplied by an amplification factor, comprising:

   means for measuring an electrical activity of a respiratory related muscle of the patient as a representation of the patient's neural respiratory drive $EAdi_{old}$;
   a first calculator (201) for multiplying the patient's neural respiratory drive $EAdi_{old}$ by an existing amplification factor $AF_{old}$ to obtain an existing predicted ventilatory assist pressure $P_{\text{target old}}$;
   a first sensor (202) for sensing an existing resulting pressure $P_{\text{result old}}$ delivered to the patient by the mechanical ventilator;
   a computerized modifier (203) of the amplification factor from the existing amplification factor $AF_{old}$ to a new amplification factor $AF_{new}$;
   a second calculator (204) for multiplying the patient's neural respiratory drive $EAdi_{old}$ by the new amplification factor $AF_{new}$ to obtain a new predicted ventilatory assist pressure $P_{\text{target new}}$;
   a second sensor (206) for sensing a new resulting pressure $P_{\text{result new}}$ delivered to the patient by the mechanical ventilator after the amplification factor has been changed from the existing amplification factor $AF_{old}$ to the new amplification factor $AF_{new}$;
   a first comparator (205) of the new predicted ventilatory assist pressure $P_{\text{target new}}$ and the existing predicted ventilatory assist pressure $P_{\text{target old}}$ to determine an anticipated change in pressure $\Delta P_{\text{target new-old}}$ that will be delivered to the patient by the mechanical ventilator;
   a second comparator (207) of the new resulting pressure $P_{\text{result new}}$ and the existing resulting pressure $P_{\text{result old}}$ to determine an actual change in pressure $\Delta P_{\text{result new-old}}$ delivered to the patient by the mechanical ventilator;
   a third comparator (208) of the anticipated change in pressure and the actual change in pressure calculating a ratio $\Delta P_{\text{resut new-old}} / \Delta P_{\text{target new-old}}$; and
   a third calculator (209) including a decision algorithm to take a decision as to increase, maintain or decrease the amplification factor depending on the value of the ratio $\Delta P_{\text{result new-old}} / \Delta P_{\text{target new-old}}$ as calculated by the third comparator (208);
   wherein the modifier (203) is adapted to automatically change the amplification factor as indicated by the decision from the decision algorithm of the third calculator (209); and
   wherein the first calculator (201), second calculator (204), first comparator (205), second comparator (207), third comparator (208) and third calculator (209) are implemented in a computer or computers.

2. A device for determining a level of ventilatory assist as defined in claim 1, wherein the first sensor (202) comprises a sensor of a pressure in patient's airways.

3. A device for determining a level of ventilatory assist as defined in claim 1 or 2, wherein the first comparator (205) of the new predicted ventilatory assist pressure $P_{\text{target new}}$ and the existing predicted ventilatory assist pressure $P_{\text{target old}}$ uses the following relation:

$$\Delta P_{\text{target new-old}} = P_{\text{target new}} - P_{\text{target old}}$$

   to show an anticipated change in pressure $\Delta P_{\text{target new-old}}$ that will be delivered to the patient by the mechanical ventilator.

4. A device for determining a level of ventilatory assist as defined in any one of claims 1 to 3, wherein the second comparator (207) of the new resulting pressure $P_{\text{result new}}$ and the existing resulting pressure $P_{\text{result old}}$ uses the following relation:

$$\Delta P_{\text{result new-old}} = P_{\text{result new}} - P_{\text{result old}}$$

to show an actual change in pressure $\Delta P_{\text{result new-old}}$ delivered to the patient by the mechanical ventilator.

5. A device for determining a level of ventilatory assist as defined in claim 4, wherein, when the amplification factor is increased and the ratio $\Delta P_{\text{resut new-old}}$ / $\Delta P_{\text{target new-old}}$ is positive, the decision algorithm of the third calculator (209) comprises means for delivering:

- a decision to further increase the amplification factor in response to the ratio $\Delta P_{\text{result new-old}}$ / $\Delta P_{\text{target new-old}}$ situated in an upper range;
- a decision to maintain the amplification factor unchanged in response to the ratio $\Delta P_{\text{result new-old}}$ / $\Delta P_{\text{target new-old}}$ situated in a middle range; and
- a decision to decrease the amplification factor in response to the ratio $\Delta P_{\text{resut new-old}}$ / $\Delta P_{\text{target new-old}}$ situated in a lower range.

6. A device for determining a level of ventilatory assist as defined in claim 4, wherein, when the amplification factor is decreased and the ratio $\Delta P_{\text{resut new-old}}$ / $\Delta P_{\text{target new-old}}$ is negative, the decision algorithm of the third calculator (209) comprises means for delivering:

- a decision to increase the amplification factor in response to a ratio $\Delta P_{\text{resut new-old}}$ / $\Delta P_{\text{target new-old}}$ situated in a lower range;
- a decision to maintain the amplification factor unchanged in response to a ratio $\Delta P_{\text{result new-old}}$ / $\Delta P_{\text{target new-old}}$ situated in a middle range; and
- a decision to further decrease the amplification factor in response to a ratio $\Delta P_{\text{resut new-old}}$ / $\Delta P_{\text{target new-old}}$ situated in an upper range.

## Patentansprüche

1. Vorrichtung zum Bestimmen eines Beatmungsgrades, der einem Patienten in Reaktion auf eine Messung eines neuralen Atemantriebs des Patienten multipliziert mit einem Verstärkungsfaktor über ein mechanisches Beatmungsgerät geliefert werden soll, umfassend:

Mittel zum Messen einer elektrischen Aktivität eines atmungsrelevanten Muskels des Patienten als eine Darstellung des neuralen Atemantriebs $EAdi_{old}$ des Patienten; einen ersten Rechner (201) zum Multiplizieren des neuralen Atemantriebs $EAdi_{old}$ des Patienten mit einem bestehenden Verstärkungsfaktor $AF_{old}$, um einen bestehenden vorhergesagten Beatmungsdruck $P_{\text{target old}}$ zu erhalten;
einen ersten Sensor (202) zum Erfassen eines bestehenden sich ergebenden Drucks $P_{\text{result old}}$, der dem Patienten über das mechanische Beatmungsgerät geliefert wird;
einen computergestützten Modifizierer (203) des Verstärkungsfaktors vom bestehenden Verstärkungsfaktor $AF_{old}$ auf einen neuen Verstärkungsfaktor $AF_{new}$;
einen zweiten Rechner (204) zum Multiplizieren des neuralen Atemantriebs $EAdi_{old}$ des Patienten mit dem neuen Verstärkungsfaktor $AF_{new}$, um einen neuen vorhergesagten Beatmungsdruck $P_{\text{target new}}$ zu erhalten;
einen zweiten Sensor (206) zum Erfassen eines neuen sich ergebenden Drucks $P_{\text{result new}}$, der dem Patienten über das mechanische Beatmungsgerät geliefert wird, nachdem der Verstärkungsfaktor vom bestehenden Verstärkungsfaktor $AF_{old}$ auf den neuen Verstärkungsfaktor $AF_{new}$ geändert wurde;
einen ersten Vergleicher (205) des neuen vorhergesagten Beatmungsdrucks $P_{\text{target new}}$ und des bestehenden vorhergesagten Beatmungsdrucks $P_{\text{target old}}$, um eine erwartete Druckänderung $\Delta P_{\text{target}}$ new-old zu bestimmen, die dem Patienten über das mechanische Beatmungsgerät geliefert werden wird; einen zweiten Vergleicher (207) des neuen sich ergebenden Drucks $P_{\text{result new}}$ und des bestehenden sich ergebenden Drucks $P_{\text{result}}$ old, um eine tatsächliche Druckänderung $\Delta P_{\text{result new-old}}$ zu bestimmen, die dem Patienten über das mechanische Beatmungsgerät geliefert wird;
einen dritten Vergleicher (208) der erwarteten Druckänderung und der tatsächlichen Druckänderung, der ein Verhältnis $\Delta P_{\text{result new-old}}$ / $\Delta P_{\text{target new-old}}$ berechnet; und
einen dritten Rechner (209), der einen Entscheidungsalgorithmus beinhaltet, um abhängig vom Wert des Verhältnisses $\Delta P_{\text{result new-old}}$ / $\Delta P_{\text{target new-old}}$, wie vom dritten Vergleicher (208) berechnet, eine Entscheidung zu treffen, ob der Verstärkungsfaktor erhöht, beibehalten oder gesenkt werden soll;

wobei der Modifizierer (203) dazu geeignet ist, den Verstärkungsfaktor automatisch wie von der Entscheidung aus dem Entscheidungsalgorithmus des dritten Rechners (209) angegeben zu ändern; und

wobei der erste Rechner (201), zweite Rechner (204), erste Vergleicher (205), zweite Vergleicher (207), dritte Vergleicher (208) und dritte Rechner (209) in einem Computer oder Computern implementiert sind.

2. Vorrichtung zum Bestimmen eines Beatmungsgrades wie in Anspruch 1 definiert, wobei der erste Sensor (202) einen Sensor eines Drucks in Luftwegen des Patienten umfasst.

3. Vorrichtung zum Bestimmen eines Beatmungsgrades wie in Anspruch 1 oder 2 definiert, wobei der erste Vergleicher (205) des neuen vorhergesagten Beatmungsdrucks $P_{target\ new}$ und des bestehenden vorhergesagten Beatmungsdrucks $P_{target\ old}$ die folgende Beziehung verwendet:

$$\Delta P_{target\ new\text{-}old} = P_{target\ new} - P_{target\ old}$$

um eine erwartete Druckänderung $\Delta P_{target\ new\text{-}old}$ zu zeigen, die dem Patienten über das mechanische Beatmungsgerät geliefert werden wird.

4. Vorrichtung zum Bestimmen eines Beatmungsgrades wie in einem der Ansprüche 1 bis 3 definiert, wobei der zweite Vergleicher (207) des neuen sich ergebenden Drucks $P_{result\ new}$ und des bestehenden sich ergebenden Drucks $P_{result\ old}$ die folgende Beziehung verwendet:

$$\Delta P_{result\ new\text{-}old} = P_{result\ new} - P_{result\ old}$$

um eine tatsächliche Druckänderung $\Delta P_{result\ new\text{-}old}$ zu zeigen, die dem Patienten über das mechanische Beatmungsgerät geliefert wird.

5. Vorrichtung zum Bestimmen eines Beatmungsgrades wie in Anspruch 4 definiert, wobei, wenn der Verstärkungsfaktor erhöht wird und das Verhältnis $\Delta P_{result\ new\text{-}old}$ / $\Delta P_{target\ new\text{-}old}$ positiv ist, der Entscheidungsalgorithmus des dritten Rechners (209) Mittel umfasst zum Liefern:

- einer Entscheidung, den Verstärkungsfaktor weiter zu erhöhen in Reaktion darauf, dass das Verhältnis $\Delta P_{result\ new\text{-}old}$ / $\Delta P_{target\ new\text{-}old}$ in einem oberen Bereich liegt;
- einer Entscheidung, den Verstärkungsfaktor unverändert beizubehalten in Reaktion darauf, dass das Verhältnis $\Delta P_{result\ new\text{-}old}$ / $\Delta P_{target\ new\text{-}old}$ in einem mittleren Bereich liegt; und
- einer Entscheidung, den Verstärkungsfaktor zu senken in Reaktion darauf, dass das Verhältnis $\Delta P_{result\ new\text{-}old}$ / $\Delta P_{target\ new\text{-}old}$ in einem unteren Bereich liegt.

6. Vorrichtung zum Bestimmen eines Beatmungsgrades wie in Anspruch 4 definiert, wobei, wenn der Verstärkungsfaktor gesenkt wird und das Verhältnis $\Delta P_{result\ new\text{-}old}$ / $\Delta P_{target\ new\text{-}old}$ negativ ist, der Entscheidungsalgorithmus des dritten Rechners (209) Mittel umfasst zum Liefern:

- einer Entscheidung, den Verstärkungsfaktor zu erhöhen in Reaktion auf ein Verhältnis $\Delta P_{result\ new\text{-}old}$ / $\Delta P_{target\ new\text{-}old}$, das in einem unteren Bereich liegt;
- einer Entscheidung, den Verstärkungsfaktor unverändert beizubehalten in Reaktion auf ein Verhältnis $\Delta P_{result\ new\text{-}old}$ / $\Delta P_{target\ new\text{-}old}$, das in einem mittleren Bereich liegt; und
- einer Entscheidung, den Verstärkungsfaktor weiter zu senken in Reaktion auf ein Verhältnis $\Delta P_{result\ new\text{-}old}$ / $\Delta P_{target\ new\text{-}old}$, das in einem oberen Bereich liegt.

**Revendications**

1. Dispositif de détermination d'un niveau d'assistance ventilatoire devant être administré à un patient par un ventilateur mécanique en réponse à une mesure d'une fonction respiratoire neurale du patient multipliée par un facteur d'amplification, comprenant :

des moyens de mesure d'une activité électrique d'un muscle lié à la respiration du patient représentant la fonction respiratoire neurale du patient $EADi_{old}$ ;

un premier calculateur (201) pour multiplier la fonction respiratoire neurale du patient $EADi_{old}$ par un facteur d'amplification existant $AF_{old}$ pour obtenir une pression d'assistance ventilatoire prédite existante $P_{target\ old}$ ;

un premier capteur (202) pour détecter une pression résultante existante $P_{result\ old}$ administrée au patient par le ventilateur mécanique ;

un modificateur informatisé (203) du facteur d'amplification du facteur d'amplification existant $AF_{old}$ à un nouveau facteur d'amplification $AF_{new}$ ;

un deuxième calculateur (204) pour multiplier la fonction respiratoire neurale du patient $EADi_{old}$ par le nouveau facteur d'amplification $AF_{new}$ pour obtenir une nouvelle pression d'assistance ventilatoire prédite $P_{target\ new}$ ;

un second capteur (206) pour détecter une nouvelle pression résultante $P_{result\ new}$ administrée au patient par le ventilateur mécanique après que le facteur d'amplification a été modifié du facteur d'amplification existant $AF_{old}$ au nouveau facteur d'amplification $AF_{new}$ ;

un premier comparateur (205) de la nouvelle pression d'assistance ventilatoire prédite $P_{target\ new}$ et de la pression d'assistance ventilatoire prédite existante $P_{target\ old}$ pour déterminer une modification anticipée de la pression $\Delta P_{target\ new-old}$ qui sera administrée au patient par le ventilateur mécanique ;

un deuxième comparateur (207) de la nouvelle pression résultante $P_{result\ new}$ et de la pression résultante existante $P_{result\ old}$ pour déterminer une modification réelle de la pression $\Delta P_{result\ new-old}$ administrée au patient par le ventilateur mécanique ;

un troisième comparateur (208) de la modification anticipée de la pression et de la modification réelle de la pression calculant un rapport $\Delta P_{result\ new-old}/\Delta P_{target\ new-old}$ ; et

un troisième calculateur (209) incluant un algorithme de décision pour prendre une décision concernant l'augmentation, le maintien ou la diminution du facteur d'amplification en fonction de la valeur du rapport $\Delta P_{result\ new-old}/\Delta P_{target\ new-old}$ tel que calculé par le troisième comparateur (208) ;

dans lequel le modificateur (203) est adapté pour modifier automatiquement le facteur d'amplification comme indiqué par la décision de l'algorithme de décision du troisième calculateur (209) ; et

dans lequel le premier calculateur (201), le deuxième calculateur (204), le premier comparateur (205), le deuxième comparateur (207), le troisième comparateur (208) et le troisième calculateur (209) sont mis en oeuvre dans un ordinateur ou des ordinateurs.

2. Dispositif de détermination d'un niveau d'assistance ventilatoire selon la revendication 1, dans lequel le premier capteur (202) comprend un capteur d'une pression dans des voies respiratoires du patient.

3. Dispositif de détermination d'un niveau d'assistance ventilatoire selon la revendication 1 ou 2, dans lequel le premier comparateur (205) de la nouvelle pression d'assistance ventilatoire prédite $P_{target\ new}$ et de la pression d'assistance ventilatoire prédite existante $P_{target\ old}$ utilise la relation suivante :

$$\Delta P_{target\ new-old} = P_{target\ new} - P_{target\ old}$$

pour montrer une modification anticipée de la pression $\Delta P_{target\ new-old}$ qui sera administrée au patient par le ventilateur mécanique.

4. Dispositif de détermination d'un niveau d'assistance ventilatoire selon l'une quelconque des revendications 1 à 3, dans lequel le deuxième comparateur (207) de la nouvelle pression résultante $P_{result\ new}$ et de la pression résultante existante $P_{result\ old}$ utilise la relation suivante :

$$\Delta P_{result\ new-old} = P_{result\ new} - P_{result\ old}$$

pour montrer une modification réelle de la pression $\Delta P_{result\ new-old}$ administrée au patient par le ventilateur mécanique.

5. Dispositif de détermination d'un niveau d'assistance ventilatoire selon la revendication 4, dans lequel, lorsque le facteur d'amplification est augmenté et que le rapport $\Delta P_{result\ new-old}/\Delta P_{target\ new-old}$ est positif, l'algorithme de décision du troisième calculateur (209) comprend des moyens pour administrer :

- une décision d'augmenter davantage le facteur d'amplification en réponse au rapport $\Delta P_{result\ new-old}/\Delta P_{target\ new-old}$ situé dans une plage supérieure ;
- une décision de maintenir le facteur d'amplification inchangé en réponse au rapport

$\Delta P_{\text{result new-old}}/\Delta P_{\text{target new-old}}$ situé dans une plage intermédiaire ; et
- une décision de diminuer le facteur d'amplification en réponse au rapport $\Delta P_{\text{result new-old}}/\Delta P_{\text{target new-old}}$ situé dans une plage inférieure.

6. Dispositif de détermination d'un niveau d'assistance ventilatoire selon la revendication 4, dans lequel, lorsque le facteur d'amplification est diminué et que le rapport $\Delta P_{\text{result new-old}}/\Delta P_{\text{target new-old}}$ est négatif, l'algorithme de décision du troisième calculateur (209) comprend des moyens pour administrer :

- une décision d'augmenter le facteur d'amplification en réponse à un rapport $\Delta P_{\text{result new-old}}/\Delta P_{\text{target new-old}}$ situé dans une plage inférieure ;
- une décision de maintenir le facteur d'amplification inchangé en réponse à un rapport $\Delta P_{\text{result new-old}}/\Delta P_{\text{target new-old}}$ situé dans une plage intermédiaire ; et
- une décision de diminuer davantage le facteur d'amplification en réponse à un rapport $\Delta P_{\text{result new-old}}/\Delta P_{\text{target new-old}}$ situé dans une plage supérieure.

**CALCULATING**

$P_{target\ old} = AF_{old} \cdot EAdi_{old}$

101

**MEASURING**

$P_{result\ old}$

102

**CHANGING (INCREASING OR DECREASING) AMPLIFICATION FACTOR TO $AF_{new}$**

103

**MEASURING**

$P_{result\ new}$

106

**CALCULATING**

$P_{target\ new} = AF_{new} \cdot Eadi_{old}$

104

**CALCULATING**

$\Delta P_{target\ new\text{-}old} = P_{target\ new} - P_{target\ old}$

105

**CALCULATING**

$\Delta P_{result\ new\text{-}old} = P_{result\ new} - P_{result\ old}$

107

**CALCULATING**

$\Delta P_{result\ new\text{-}old}\ /\ \Delta P_{target\ new\text{-}old}$

108

100

**DECISION TO INCREASE OR DECREASE AMPLIFICATION FACTOR (AF)**

109

FIGURE 1

FIGURE 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1204439 B1

- US 5671752 A, Sinderby